(19) Europäisches Patentamt
European Patent Office
Office européen des brevets

(11) Numéro de publication: 0 136 947
A2

# (12) DEMANDE DE BREVET EUROPEEN

(21) Numéro de dépôt: 84401933.1

(22) Date de dépôt: 27.09.84

(51) Int. Cl.[4]: C 08 B 37/00
A 61 K 37/20, A 61 K 39/395

(30) Priorité: 30.09.83 FR 8315683

(43) Date de publication de la demande: 10.04.85 Bulletin 85/15

(84) Etats contractants désignés: AT BE CH DE FR GB IT LI LU NL SE

(71) Demandeur: INSTITUT PASTEUR
25-28, rue du Docteur Roux
F-75724 Paris Cedex 15(FR)

(71) Demandeur: Etablissement Public dit: CENTRE NATIONAL DE LA RECHERCHE SCIENTIFIQUE (CNRS)
15, Quai Anatole France
F-75007 Paris(FR)

(72) Inventeur: Lange, Marc
62, rue Tiquetonne
F-75002 Paris(FR)

(72) Inventeur: Cazenave, Pierre
115, avenue du Maine
F-75014 Paris(FR)

(72) Inventeur: Leguern, Christian
11, rue du Moulin de la Roche
F-94250 Gentilly(FR)

(74) Mandataire: Phélip, Bruno et al,
c/o Cabinet Harlé & Phélip 21, rue de la Rochefoucauld
F-75009 Paris(FR)

## (54) Lipopolysaccharides conjugées, leur procédé de préparation, leur application comme agents immunogéniques.

(57) L'invention concerne la préparation de lipopolysaccharides modifiés porteurs de fonctions cyanates, et leur conjugaison avec des molécules porteuses de groupes nucléophiles, tels que peptides, protéines, acides nucléiques, glycopeptides, glycoprotéines, haptènes et molécules organiques de synthèse.

Application de ces conjugués au déclenchement de la sécrétion d'anticorps spécifiques, dirigés contre la molécule porteuse de groupes nucléophiles, par des lymphocytes B, in vivo comme in vitro, et à l'obtention d'hybridomes sécréteurs de ces anticorps.

EP 0 136 947 A2

Croydon Printing Company Ltd.

"Lipopolysaccharides conjugués, leur procédé de préparation, leur application comme agents immunogéniques"

La présente invention concerne la conjugaison de lipopolysaccharides, ou LPS, à diverses molécules à groupes nucléophiles, par l'intermédiaire de fonctions cyanates, les conjugués obtenus notamment avec des protéines, des peptides, des acides nucléiques, des glycoprotéines, des glycopeptides et des molécules organiques de synthèse et leur utilisation pour l'immunostimulation in vivo ou in vitro des lymphocytes B en vue de la sécrétion d'anticorps spécifiques.

On sait que les lipopolysaccharides extraits des parois bactériennes stimulent la mitose des lymphocytes B et la sécrétion des anticorps par ces cellules. BRADLEY S.A. a fait une revue de ces propriétés dans Ann.Rev. Microbiol. 33, 67 (1979).

COUTINHO et coll. dans J. Exp. Med. 139,74 (1974) ont décrit la préparation d'un conjugué de lipopolysaccharide (ou LPS) et d'hydroxy-4 dinitro-3,5 phényl par action du diazoïque correspondant avec un LPS bactérien. Ils ont montré que ce conjugué présent à haute concentration dans le milieu de culture, stimule in vitro de façon polyclonale les cellules B, tandis qu'à faible concentration il induit une réponse spécifique anti-hydroxy-4 dinitro-3,5 phényl. Cette méthode implique l'utilisation d'une molécule porteuse d'une fonction susceptible de réagir sur le LPS ce qui en limite les possibilités. En outre les quantités des réactifs mis en jeu sont importantes, de l'ordre de 10 à 100 mg.

PRIMI et coll. dans J. Immunol. 129 3 (1982) ont montré que le milieu réactionnel résultant de l'action du glutaraldéhyde sur un mélange de lipopolysaccharide et d'anticorps monoclonal en milieu aqueux, peut induire une réponse immunitaire in vivo et in vitro. Un tel procédé classique de couplage, ne peut être réalisé qu'entre un LPS et une autre macromolécule, telle qu'une immunoglobuline, et nécessite de travailler sur des quantités importantes d'antigènes, de l'ordre du mg. Par ailleurs les essais de PRIMI ont été effectués avec des milieux réactionnels de composition mal définie, notamment parce que les réactions de couplage utilisées sont difficiles à contrôler, et on ne saurait conclure sur la nature exacte des agents actifs.

On a trouvé maintenant un nouveau procédé de conjugaison des lipopolysaccharides à toutes sortes de molécules porteuses de groupes nucléophiles, ou MGN, procédé simple et reproductible qui permet d'obtenir un conjugué LPS/MGN parfaitement défini, où les diverses parties sont liées par des liaisons covalentes,et conservant les propriétés immunogéniques du LPS de départ. Ce procédé n'implique plus, comme avec la méthode de conjugaison de COUTINHO et coll. la réaction d'un antigène porteur de groupements qui peuvent réagir sur les LPS mais celle d'un LPS modifié, polyvalent, pouvant réagir sur des très nombreuses molécules qui n'ont pas besoin d'être préalablement activées.

Les LPS modifiés et activés de l'invention sont porteur de fonction cyanates; la cyanation des LPS est effectuée, par exemple par action du bromure de cyanogène ou par tout autre système générateur d'ions $CN^+$.

On sait que les LPS sont composés de trois sous-unités : une unité, le lipide A, qui est à l'origine des propriétés immunostimulantes des LPS, liée à deux sous-unités polysaccharidiques. Par le procédé de l'invention, seule la structure polysaccharidique sera modifiée, selon le schéma suivant : (les OH sont ceux normalement présents sur le LPS)

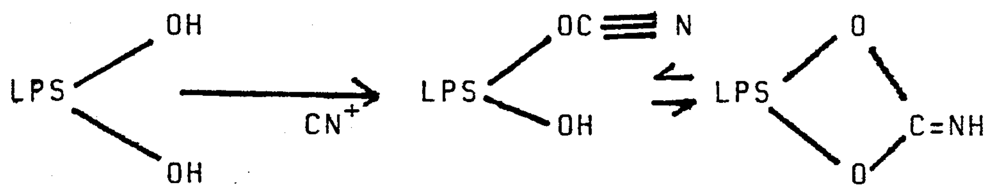

Les LPS activés de l'invention peuvent porter un nombre variable de groupes cyanates sur le polysaccharide , en fonction des conditions opératoires utilisées. Dans une réalisation préférée du procédé de l'invention, on limite la polymérisation des LPS, pour conserver une activité immunogénique au conjugué LPS/MGN, en effectuant la cyanation du LPS en solution aqueuse basique, par action de CNBr en solution dans l'acétonitrile à des concentration, dose et pour des durées de réaction bien définies.

Dans le procédé de l'invention, à une température comprise entre 0ºC et la température ambiante, on ajoute une quantité donnée de CNBr,en solution dans un solvant organique, à une solution aqueuse basique de LPS; après un temps de l'ordre de quelques minutes, entre 1 à 20 minutes , on arrête la réaction par acidification du milieu et on isole les LPS activés formés en les précipitant par addition d'un solvant organique approprié

dans le milieu.

On opère de préférence à 4°C avec du bromure de cyanogène, en solution 1M dans l'acétonitrile et du LPS en solution aqueuse basique de pH inférieur à 10 environ et de concentration 0,2 à 10 mg/ml; on arrête la réaction après 3 minutes environ et isole immédiatement le LPS modifié du milieu réactionnel.

Le produit obtenu est ensuite mis en suspension dans une solution diluée d'acide chlorhydrique de pH compris entre 2,5 et 3,5 puis dispersé, par exemple par passage à travers une aiguille de faible diamètre (de 0,1 à 0,5 mm environ), pour isoler du LPS activé conservant une partie de l'activité mitogénique du LPS de départ; en effet, par ce traitement, on réduit avantageusement le degré de polymérisation du LPS activé.

Les LPS modifiés, obtenus par le procédé selon l'invention comportent au plus 200 micromoles environ de groupes cyanates par gramme de LPS de départ, de préférence 100 micromoles environ; la quantité de groupes cyanates fixés peut être mesurée par la méthode colorimétrique décrite par KOHN et coll. dans Biochem.Biophy. Res Comm. <u>87</u> 7 (1978). Les LPS activés de l'invention peuvent être conservés plusieurs mois vers - 20°C en suspension dans une solution aqueuse d'acide chlorhydrique $10^{-3}$N environ, sans dégradation appréciable, ce qui est un autre avantage de l'invention.

Etant donné la grande réactivité des fonctions cyanates, les LPS modifiés peuvent être facilement couplés à de nombreuses molécules porteuses de groupes nucléophiles, tels que des peptides, des protéines, des acides nucléiques, des glycoprotéines et glycopeptides des haptènes comme les

hormones et les prostaglandines ou des molécules organiques de synthèse. Les groupes nucléophiles susceptibles de réagir sur les LPS activés sont nombreux et certains réagiront même dans des conditions très douces de couplage , ceci permettra par exemple, de ne pas dénaturer les protéines. Ces groupes comprennent notamment les hydroxyles des alcools et phénols, les thiols, les fonctions amines primaines et secondaires.

La réaction de couplage du LPS modifié selon l'invention et du composé porteur des groupes nucléophiles est effectuée dans une solution aqueuse tamponnée, à un pH basique compris entre 8 et 9,5, sous agitation et à une température comprise entre la température ambiante et 0°C. Les quantités relatives des deux produits à coupler sont essentiellement fonction de leur structure et du résultat recherché et l'homme du métier sera à même de les déterminer. Pour un rendement efficace de couplage, on les mélange en général dans un rapport minimal de 0,25 en poids de MGN par rapport au poids de LPS de départ. La réaction s'effectue en quelques heures à température ambiante, ou en une journée aux environs de 4°C. Le conjugué formé est isolé du milieu réactionnel par centrifugation, opération que l'on répétera, après remise en suspension dans une solution aqueuse tamponnée, jusqu'à obtenir un conjugué débarrassé des molécules à groupes nucléophiles n'ayant pas réagi. Aussi, un autre avantage de l'invention est la simplicité et le rendement de l'opération d'isolement du conjugué du milieu réactionnel; on sait que l'isolement est déterminant pour la stimulation des lymphocytes par les conjugués de l'invention, et il est important que la séparation par centrifugation permette d'isoler 100% du conjugué formé actif.

Le conjugué ainsi obtenu peut comporter des fonctions cyanates n'ayant pas réagi; aussi afin de le stabiliser et d'éviter toute réaction secondaire gênante des fonctions cyanates lors des applications ultérieures, on traitera, par exemple par un excès de lysine ou d'éthanolamine le conjugué après l'avoir dépolymérisé dans l'eau par l'action d'ultra-sons.

Selon un aspect préféré de l'invention, les conjugués sont utilisés comme agents immunogéniques in vivo ou in vitro et les LPS modifiés convenant particulièrement pour cette application sont ceux pour lesquels on a 90 à 110 µmoles de fonctions cyanates par gramme de LPS de départ. Les conjugués LPS/MGN déclenchent la sécrétion d'anticorps spécifiques par les lymphocytes B, in vivo ou in vitro et les anticorps produits sont essentiellement dirigés contre la molécule MGN qui participe au conjugué.

Pour développer une réaction immunitaire in vivo, on injecte aux animaux le conjugué LPS/MGN en solution dans un tampon physiologique. L'utilisation, in vivo, des conjugués selon l'invention est particulièrement intéressante lorsqu'on ne dispose que d'une quantité d'antigène très faible (inférieure à 10 µg par souris de 20g). La réaction immunitaire spécifique ne serait alors pas provoquée par injection de cette quantité d'antigène seul, mais est parfaitement décelable avec la même quantité administrée sous forme conjuguée aux LPS. Ainsi la conjugaison de MGN selon l'invention, du fait du grand potentiel immunogénique des conjugués préparés, permet d'obtenir une

réaction immunitaire spécifique, nette et utile, même avec des quantités très faibles de MGN, ce qui est particulièrement intéressant lorsque l'-"antigène" est rare et coûteux.

Une autre propriété des conjugués selon l'invention est leur action mitogénique sur les cellules B in vitro. Les LPS modifiés et les conjugués qui en dérivent n'ont une action mitogénique que si on les soumet à un court traitement par ultrasons, quileur permet d'avoir une activité mitogénique comparable à celle des LPS natifs. Pour déclencher la sécrétion d'anticorps par les lymphocytes B in vitro en culture, on met les cellules, cultivées dans un milieu standard,en présence d'une quantité définie de conjugué LPS/MGN mitogénique ce quine déclenche la réponse immunitaire que contre le MGN conjugué. Si on introduit dans le milieu de culture à base de RPMI-1640 complet (commercialisé par GIBCO) les conjugués à des concentrations allant de $10^{-1}$ à $10^{-5}$ $\mu$g/ml, la sécrétion d'anticorps est décelable dès le cinquième jour de la culture.

Selon un autre aspect de l'invention, les cellules B cultivées en présence du conjugué LPS/MGN sont utilisées comme partenaires dans un procédé classique de fusion des cellules, par exemple celui décrit par KOHLER et MILSTEIN dans Nature 256 p. 495 (1975). On obtient ainsi des hybridomes sécréteurs d'anticorps spécifiques du MGN présent dans le conjugué. Cette technique, dont toutes les étapes se déroulent in vitro, permet l'obtention d'anticorps monoclonaux à partir de lymphocytes B stimulés in vitro avec de faibles quantités de MGN. Elle permet d'envisager l'exploration par les anticorps monoclonaux de nombreux MGN pondéralement peu représentés, rares et coûteux; les anticorps monoclonaux obtenus avec les conjugués LPS/MGN peuvent être utilisés comme réactifs de laboratoire et inclus notamment dans des coffrets de diagnostic classiques.

Enfin le conjugué LPS/MGN, éventuellement associé à d'autres substances actives, et mis sous forme pharmaceutiquement acceptable est utilisable en médecine humaine et vétérinaire.

Dans ce qui suit, des exemples non limitatifs sont donnés à titre d'illustration de différents aspects de l'invention. Pour étudier l'activité mitogénique sur les cellules B en culture, des conjugués, on a mesuré l'incorporation de thymidine marquée au tritium dans des cultures de cellules de rates en présence de concentrations variables de divers conjugués préparés selon l'invention. On a comparé l'action des conjugués avec celle des LPS natifs, ou même effectué l'essai en l'absence de tout agent de stimulation. L'expérience a été effectuée comme suit : 0,2 ml de culture cellulaire (5x $10^5$ cellules par ml d'un milieu à base de RPMI 1640 complémenté) dans les micropuits d'une plaque sont incubés 72heures à 37°C dans une atmosphère à 7% de $CO_2$. On introduit alors 3,7 x $10^4$ cpm de thymidine tritiée d'activité spécifique 1mCi/mmole dans chacun des puits et 4 heures après on mesure l'incorporation de la thymidine dans les cellules.

Pour étudier l'activité immunogénique des conjugués LPS/MGN, qui se traduit par une présence d'anticorps spécifiques du MGN dans le sérum des animaux auxquels a été injecté du conjugué ou dans

les milieux des cultures de lymphocytes où le conjugué a été introduit en quantité définie, on a effectué des essais d'agglutination entre le sérum ou les surnageants des cultures à étudier et contenant les anticorps, et des préparations contenant des érythrocytes de mouton couplés aux MGN. Les couplages aux érythrocytes ont été effectués soit par action de $CrCl_3$, comme décrit par PRIMI D et CAZANAVE P.A. dans J. Immunol. 129 3 (1982) soit parla méthode décrite par RITTENBERG et coll. dans Proc. Soc. Exp. Biol. Med. 132 175 (1969). On a fait une série de dilutions du sérum ou du surnageant à tester dans une solution physiologique contenant 3% de sérum de veau foetal, et on les a réparti dans des plaques de microtitrage à fond en V puis 25 µl de préparation à 0,5% d'érythrocytes couplés au MGN ont été ajoutés dans chaque puits et l'ensemble agité. On a attendu 2 heures avant de déterminer visuellement le titre de l'hémagglutination.

EXEMPLE 1

Préparation de LPS modifiés

Une solution de 20 mg de lipopolysaccharide W provenant de *Salmonella Thyphimurium* commercialisé par DIFCO Laboratories (Detroit-USA) dans 5 ml d'eau distillée est agitée et refroidie à 4°C. On ajoute alors simultanément 0,2 ml de solution aqueuse de carbonate de sodium 2M et 0,2 ml d'une solution de CNBr dans l'acétonitrile anhydre à 0,95 M. Après 3 minutes, on arrête la réaction par addition de 0,8 ml d'une solution aqueuse d'acide chlorhydrique 1N, puis on ajoute immédiatement 50 ml d'acétone, préalablement refroidie à 4°C et contenant 0,1% de solution de HCL 1N pour précipiter

les LPS modifiés. Ceux-ci sont isolés par centrifugation à 13000 rpm dans une centrifugeuse SORVAL ® RC5B rotor SS 34 pendant 5 minutes. Le LPS modifié isolé est ensuite lavé deux fois avec de l'acétone et séché sous vide.

On le met ensuite en suspension à raison de 10 mg dans 2 ml de solution aqueuse d'acide chlorhydrique $10^{-3}$N, pour le conserver à une température d'environ - 20°C.

Ces composés sont plus ou moins polymérisés et avant couplage avec une molécule à groupes nucléophiles, on pulvérise la suspension chlorhydrique en la faisant passer à travers une aiguille de 0,2 mm de diamètre.

Le LPS modifié ainsi obtenu a environ 100 µmoles de fonctions cyanate par gramme de LPS. Si on effectue la cyanation avec la quantité double de CNBr, on obtient un produit comportant 160 µmoles environ de fonctions cyanates par gramme.

EXEMPLE 2

Conjugués de LPS

a) Macrométhode

On centrifuge à 13000 rpm dans la même centrifugeuse, que ci-dessus, pendant 5 minutes, la suspension chlorhydrique préparée selon l'exemple 1 et le LPS modifié est récupéré après élimination du surnageant. On le mélange alors avec le MGN en solution dans un tampon à pH compris entre 8 et 9,5. Les tampons 0,1M de phosphate, carbonate et borate conviennent, à l'exclusion des tampons contenant des fonctions susceptibles de réagir sur les cyanates tels que ceux contenant des composés ayant des groupes nucléophiles. On laisse la

réaction évoluer 3 heures à 20°C, ou encore 18 heures à 4°C, de préférence sous agitation, avant de séparer le conjugué LPS/MGN par centrifugation à 13000 rpm, pendant au moins 15 minutes. Dans ces conditions, on élimine aussi dans le surnageant l'antigène de départ; toutefois dans certains cas, il peut être nécessaire de répéter plusieurs fois cette opération de centrifugation; pour cela le surnageant est éliminé après chaque centrifugation et un même volume du même tampon que celui pour la réaction de couplage est réintroduit dans le tube avant une nouvelle centrifugation.

Si nécessaire, on soumet ensuite le conjugué ainsi obtenu à l'action d'un excès de lysine en solution aqueuse : après dissolution du conjugué dans l'eau par action brève d'ultrasons et obtention d'une solution de concentration environ 1 mg/ml, on ajoute une quantité suffisante de lysine pour que la solution finale soit environ $10^{-2}$M. On laisse agir de 30 à 120 minutes, puis centrifuge pour isoler le conjugué ne comportant plus de groupes cyanates libres.

On a préparé des conjugués de LPS avec des protéines marqués à l'$^{125}$I, par action de la chloramine T comme décrit par SONODA et coll. dans Immunochemistry 7 885 (1970), afin de faciliter la détermination de la quantité de protéine fixée par unité de poids de lipopolysaccharide en fonction des quantités relatives de LPS et protéine.

La figure 1 donne des résultats obtenus avec 1 mg de $\beta$-galactosidase d'*Escherichia coli*, de cytochrome C de coeur de cheval (commercialisés par Sigma Chem. Co.- St- Louis - USA) et de protéine F6 (51), l'immunoglobuline monoclonale anti-M 460 décrite par BUTTIN et coll. dans Curr.

Topics. Microbiol. Immun. 81 127 (1978). On a utilisé 0,1 ml de solution tampon contenant des quantités variables de protéines (de 0,5 à 10 mg/ml environ).

La figure 2 montre la quantité de β-galactosidase fixée par gramme de LPS modifié préparé selon l'exemple 1, en fonction de la quantité de protéine ajoutée et de la concentration de la solution.

b) Microméthode

La suspension de LPS activé dans l'acide chlorhydrique $10^{-3}$N est soumise à un traitement par ultrasons jusqu'à solubilisation et obtention d'une solution de concentration 1 mg/ml environ. On prélève alors environ 5 µl de cette solution et y ajoute de 5 à 10 µg du MGN en solution dans le tampon. La suite de la réaction et le traitement sont effectués comme décrit pour la macrométhode. L'activité mitogénique des conjugués ainsi préparés, a été mesurée après les avoir soumis à un bref traitement par ultrasons. Pour cela, on a soumis la suspension de conjugué à des ultrasons d'amplitude 5 à 20 µ, pendant 5 à 15 secondes.

Dans le tableau 2, les résultats sur l'activité mitogéniquede conjugués réactivés par ultrasons et du LPS de départ sur des cultures cellulaires, dans le milieu duquel ils se trouvaient à diverses concentrations, sont indiqués.

EXEMPLE 3

Immunisation des souris

Des groupes de 3 souris Balb/c sont immunisés par une injection intrapéritonéale de 5 µg d'un agent immunogénique, dissous dans le même tampon que celui de la réaction de couplage. Le 7ème jour, on prélève le sang au niveau du plexus rétro-

orbitalaire pour déterminer par hémagglutination, comme décrit précédemment, le titre en anticorps du sérum.

Le tableau 1 donne les résultats obtenus, exprimés en logarithmes décimaux, pour différents agents immunogèniques : des molécules à groupes nucléophiles (MGN), des LPS purs ou des conjugués LPS/MGN. On constate que les lypopolysaccharides non conjugués, comme les antigènes non conjugués n'ont aucune activité.

On a obtenu des résultats comparables en injectant 1 µg des mêmes agents immunogèniques et en effectuant les prélèvements sanguins seulement 15 jours après l'injection.

## TABLEAU 1

| Agents immunogéniques | Erythrocytes | | | |
|---|---|---|---|---|
| | Seuls | Couplés à TNP ** | Couplés à β -galactosidase | Couplés*** à F6(51) |
| LPS | 2 | 1 | 1 | 2 |
| DNP lysine * | 1 | 1 | 2 | 2 |
| DNP lysine/LPS | 2 | 13 | 3 | 1 |
| β -galactosidase | 1 | 1 | 2 | 1 |
| β - galactosidase/LPS | 2 | 1 | 24 | 1 |
| F6(51) | 2 | 2 | 1 | 2 |
| F6(51)/LPS | 2 | 1 | 2 | 14 |

* DNP : ε N- (dinotrophényl)lysine

** TNP : acide trinitrobenzène sulfonique

*** F6 (51):immunoglobuline préparée par Buttin et coll. (référence ci-dessus)

100 µg de β-galactosidase ou de F6(51) sont fixés par mg de conjugué où 40 µmoles de DNP lysine ont réagi par g de LPS.

Lorsque les conjugués sont préparés à partir de faibles quantités de protéines, par la microméthode selon l'exemple 1, il suffit de 5 à 10 µg de protéines de départ pour déclencher une réaction spécifique chez ces animaux par injection du conjugué préparé selon l'exemple 2 .

EXEMPLE 4

Induction par les conjugués de la sécrétion d'anticorps par des cellules en culture.

On prélève les rates de souris Balb/c et les dilacère dans une solution saline isotonique tamponnée. On lave les cellules ensuite 2 fois par 50 ml de cette même solution, puis on les met en suspension à raison de $10^7$ cellules par ml dans la même solution et les traite successivement par un anticorps monoclonal anti-Thy-1-2 et par du complément, selon le procédé décrit par PRIMI et coll. Les cellules sont ensuite mises en culture à raison de $5 \times 10^5$ cellules/ml dans un milieu RPMI 1640, complémenté avec de la glutamine ($2 \times 10^{-3}$M), des antibiotiques (1000 UI/ml), 10% de sérum de veau foetal et du mercaptoéthanol à une concentration de $2 \times 10^{-5}$ M.

Les figures 3 et 4 donnent les résultats obtenus pour des lots de 12 cultures de 0,2 ml chacune; pour chaque lot ont été introduits dans le milieu des quantités variables d'inducteurs de nature différente : conjugués de lipopolysaccharides et protéines ou LPS purs.Ne sont pas représentés les essais effectués avec des conjugués n'ayant pas d'activité mitogénique , ceux qui in vitroont toujours donné des résultats négatifs, c'est-à-dire qu'il n'y a pas eu de sécrétion d'anticorps dans les cultures où ils ont été introduits.

La figure 3 correspond à des conjugués LPS/ $\beta$-galactosidase, présentant des rapports en poids de protéine fixée au LPS, différents (courbe 1 : 200 $\gamma$ de $\beta$-gal/mg LPS; courbe 2 : 100 $\gamma$/mg; 3 : 3,25 $\gamma$/mg; courbe 4 : 0 $\gamma$/mg et LPS non traité). Les surnageants des cultures ont été prélevés 10 jours après l'addition du LPS conjugué ou non, et soumis à une recherche de la présence d'anticorps par héméagglutination avec des érythrocytes couplés à de la $\beta$-galactosidase. On a noté le pourcentage de résultats positifs, c'est-à-dire le % des cultures parmi les 12 donnant une agglutination. La courbe 5 correspond à la même culture que la courbe 1, mais l'agglutination a été étudiée avec des érythrocytes couplés au TNP.

La figure 4 correspond à des cultures traitées par un conjugué résultant du couplage de LPS et DNP-lysine, selon le procédé des exemples 1 et 2. l'agglutination a été étudiée avec les érythrocytes couplés au TNP ▲—▲ ou couplés à la $\beta$-galactosidase ▲---▲ .

Sur cette même figure, sont indiqués les résultats obtenus avec des cultures traitées par un conjugué F6(51)/LPS; l'agglutination a été étudiée pour des érythrocytes couplés à F6 (51) ■—■ ou au TNP ●-----● .

TABLEAU 2

| Concentration de l'agent mitogénique dans le milieu de culture (µg/ml) | LPS pur | Conjugué β-gal/LPS | | Conjugué LPS/DNP | Conjugué LPS/lysine |
|---|---|---|---|---|---|
| | | a | b | c | d |
| 100 | 28634±990 | 17602±937 | 16633±1058 | 17316±1135 | 16907±1253 |
| 50 | 14836±971 | 10560±962 | 10342±906 | 11130±991 | 10996±1033 |
| 5 | 7665±738 | 6512±612 | 6061±707 | 5982±512 | 5897±604 |
| 0,5 | 2981±340 | 2811±417 | 2567±416 | 3120±498 | 3040±312 |
| 0,05 | 941±186 | 967±213 | 888±227 | 1111±175 | 1001±204 |

Les résultats sont exprimés en coups par minutes (cpm)

a - conjugué où 100 µg de β-galactosidase sont fixés par mg de LPS

b - 50 µg β-gal/mg LPS

c - conjugué où 40 µmoles de dinitrophényl lysine sont fixés par 1 g de LPS

d - LPS activé et dans lequel les groupes cyanates ont tous été bloqués par de la lysine

Dans les cultures non traitées on trouve environ 1000 coups par minute.

REVENDICATIONS

1. Lipopolysaccharides modifiés, chimiquement réactifs, caractérisés en ce qu'ils sont porteurs de groupes cyanates et sont représentés par la formule :

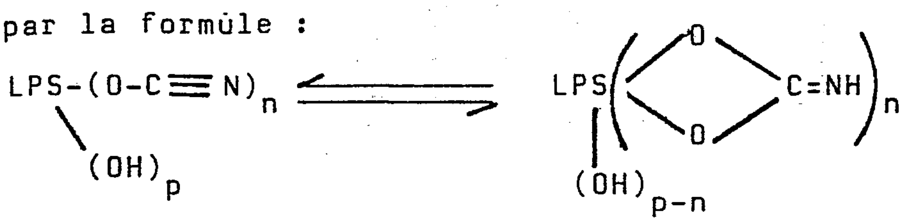

telle que $LPS(OH)_{p+n}$ représente le lipopolysaccharide de départ.

2. Procédé de préparation de lipopolysaccharides modifiés selon la revendication 1, caractérisé en ce que l'on fait réagir sur le lipopolysaccharide du bromure de cyanogène ou un système générateur d'ions $CN^+$.

3. Procédé de conjugaison de lipopolysaccharides à des molécules portant des groupes nucléophiles, caractérisé en ce que l'on fait réagir les lipopolysaccharides modifiés selon la revendication 1 sur des molécules portant des groupes nucléophiles.

4. Procédé selon la revendication 3, caractérisé en ce que les molécules portant des groupes nucléophiles sont choisies parmi les peptides, les protéines, les acides nucléiques, les glycoprotéines, les glycopeptides, les haptènes et les molécules organiques de synthèse.

5. Procédé de conjugaison selon l'une des revendications 3 ou 4, caractérisé en ce que la réaction a lieu en milieu basique à pH compris entre 8 et 9,5.

6. Conjugués de lipopolysaccharides et de molécules porteuses de groupes nucléophiles, caractérisés en ce qu'ils sont obtenus par le procédé selon l'une quelconque des revendications 3 à 5.

7. Agent de stimulation immunitaire spécifique des lymphocytes B in vivo et in vitro, caractérisé en ce qu'il comprend un conjugué selon la revendication 6.

8. Application de l'agent de stimulation selon la revendication 7 à l'immunisation animale et humaine.

9. Composition pour l'immunisation in vivo contre une molécule porteuse de groupes nucléophiles, caractérisée en ce qu'elle comprend un conjugué selon la revendication 6, associé à un véhicule pharmaceutiquement acceptable.

10. Procédé d'obtention in vivo d'anticorps spécifiques d'une molécule à groupe nucléophiles, caractérisé en ce qu'il consiste à injecter une composition selon la revendication 9.

11. Agents mitogéniques des lymphocytes B, caractérisés en ce qu'ils sont préparés par le procédé de couplage selon l'une quelconque des revendications 3 à 5, et queledit procédé est suivi d'un traitement par ultra-sons.

12. Procédé de préparation in vitro d'anticorps spécifiques, caractérisé en ce que les lymphocytes B sont cultivés en présence d'agents mitogéniques selon la revendication 11.

13. Anticorps spécifiques de molécules à groupes nucléophiles, caractérisés en ce qu'ils sont préparés par le procédé selon la revendication 12.

14. Application des conjugués de lipopolysaccharides selon la revendication 6 ou la revendication 11 à la préparation de lymphocytes B qui seront fusionnés pour donner les hybridromes secréteurs d'anticorps spécifiques de molécules porteuses de groupes nucléophiles.

15. Anticorps monoclonaux, caractérisés en ce qu'ils sont obtenus à partir des hybridomes selon la revendication 14.

FIG. 1/4

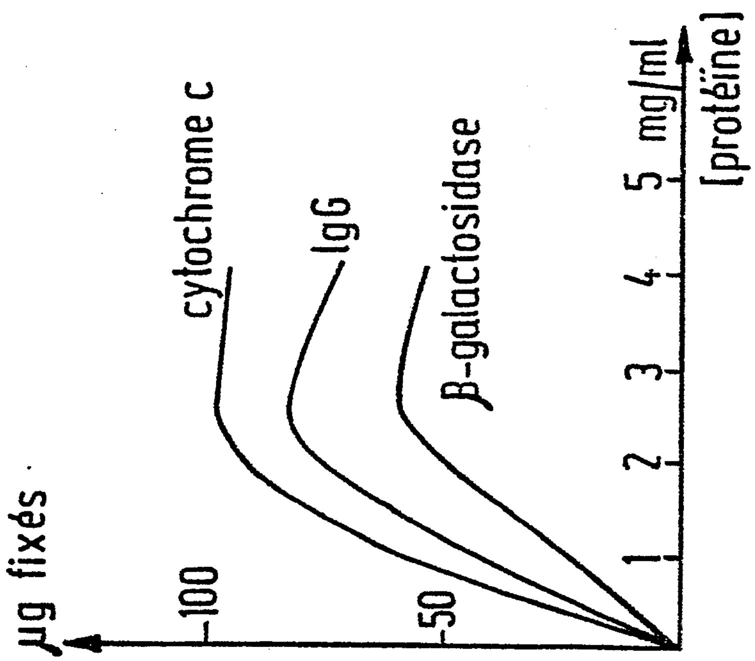
µg fixés
100
50
cytochrome c
IgG
β-galactosidase
1
2
3
4
5
mg/ml
[protéine]

FIG. 2/4

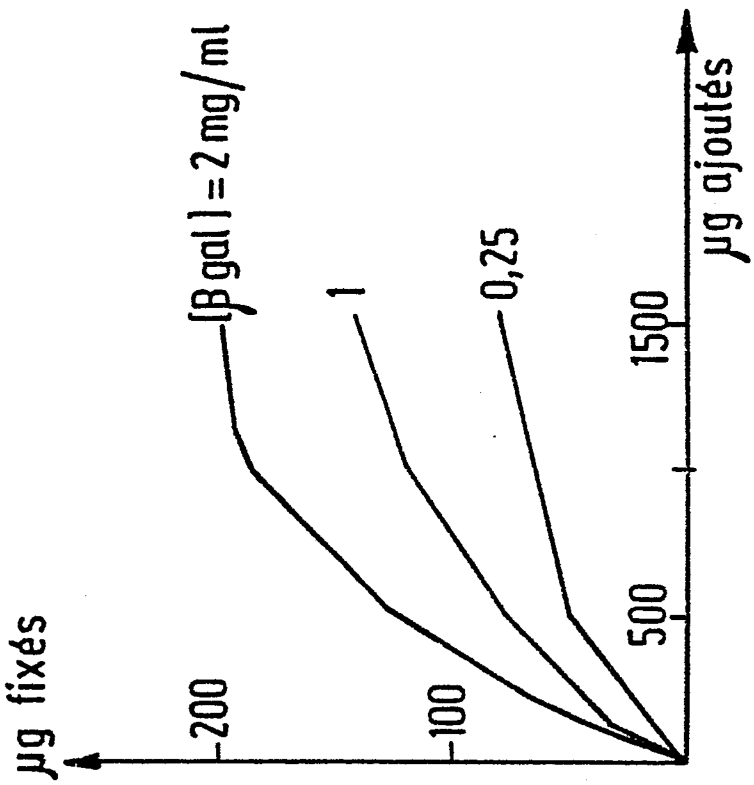
µg fixés
200
100
[β gal] = 2 mg/ml
1
0,25
500
1500
µg ajoutés

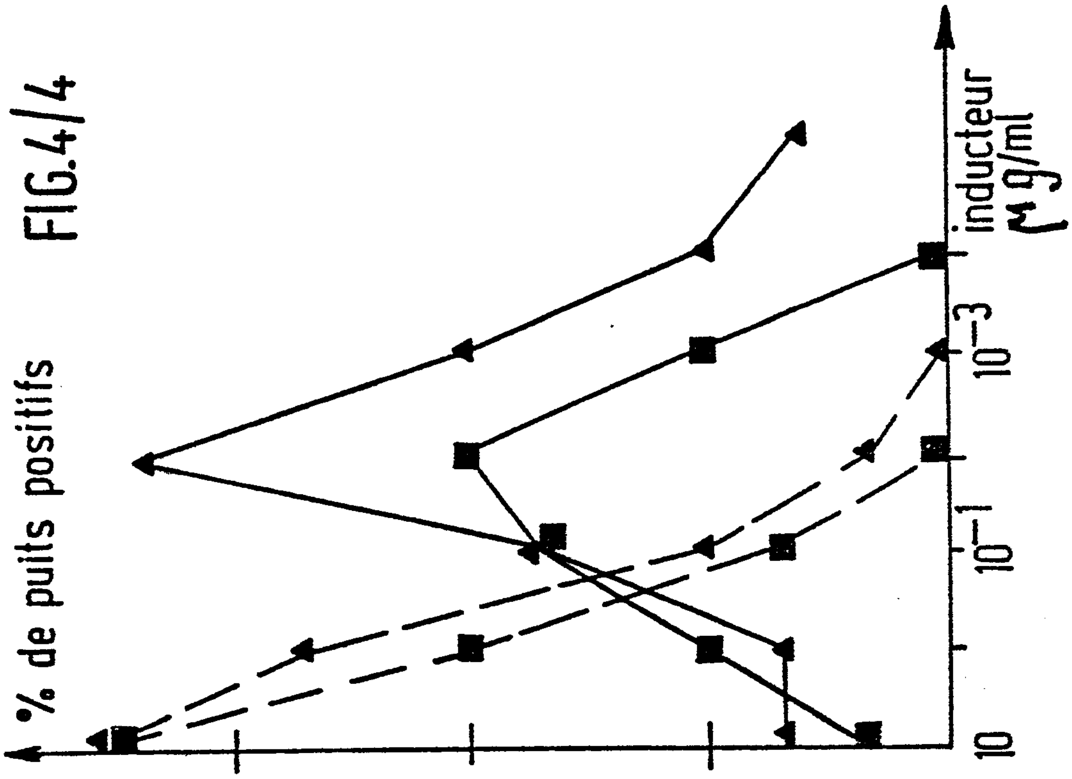
% de puits positifs
inducteur
µg/ml
10
$10^{-1}$
$10^{-3}$

FIG.4/4

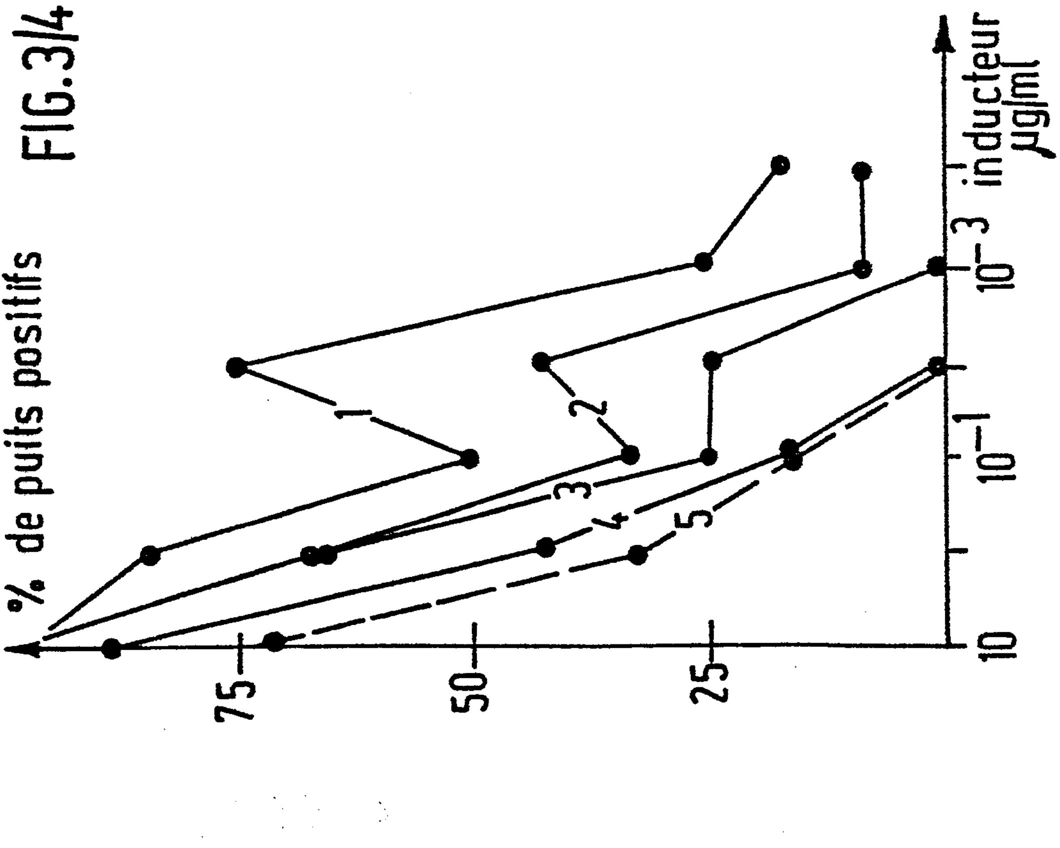
% de puits positifs
inducteur
µg/ml
75
50
25
10
$10^{-1}$
$10^{-3}$
1
2
3
4
5

FIG.3/4